# EUROPEAN PATENT SPECIFICATION

(11) **EP 1 416 942 B1**
(45) Date of publication and mention of the grant of the patent: **12.12.2007**
(21) Application number: 02726732.7
(22) Date of filing: 10.04.2002
(51) Int. Cl.: A61K 31/785, A61P 3/00, A61K 45/06

(54) **AMINE POLYMERS FOR TREATING GOUT AND BINDING URIC ACID**
AMINPOLYMERE ZUR BEHANDLUNG VON GICHT UND ZUR SENKUNG DES HARNSÄUREGEHALTES
AMINOPOLYMERES POUR TRAITER LA GOUTTE ET POUR FIXER L'ACIDE URIQUE

(30) Priority: 18.04.2001 US 284445 P; 13.07.2001 US 305567 P
(43) Date of publication of application: 12.05.2004
(73) Proprietor: GENZYME CORPORATION, Cambridge, MA 02142 (US)
(72) Inventor: HOLMES-FARLEY, Stephen, Randall, Arlington, MA 02174 (US); BURKE, Steven, K., Sudbury, MA 01776 (US)
(74) Representative: Tauchner, Paul
(86) International application number: PCT/US2002/011492
(87) International publication number: WO 2002/085381

(56) References cited:
- EP-A- 0 303 296
- WO-A-98/17707
- WO-A-02/085380
- US-B1- 6 177 478
- NEPHROLOGY, DIALYSIS, TRANSPLANTATION, vol. 14, 1999, pages 2907-2914, XP008049272 ENGLAND
- JONES R: "COLESEVELAM HYDROCHLORIDE GELTEX PHARMACEUTICALS" CURRENT OPINION IN CARDIOVASCULAR, PULMONARY AND RENAL INVESTIGATIONAL DRUGS, vol. 2, 2000, pages 284-291, XP001097580 LONDON

## Description

The prevalence of gout is approximately 1.3 to 3.7 percent of the general population. Individuals suffering from gout excrete approximately 40 percent less uric acid, the final breakdown product of purine degradation, than nongouty individuals for any given plasma urate concentrations. Hyperuricemia, a condition which precedes gout, can result from increased production or decreased excretion of uric acid, or from a combination of the two processes. In an individual with hyperuricemia, plasma and extracellular fluids are supersaturated with urate (a serum uric acid level greater than 8.5 mg/dL at baseline), and crystal deposition in tissue is likely to occur, resulting in the clinical manifestations of gout. Acute gout typically results following a prolonged period in which excessive amounts of uric acid and urate are present in serum.

Gout includes a group of disorders such as painful attacks of acute, monarticular, inflammatory arthritis, deposition of urate crystals in joints, deposition of urate crystals in renal parenchyma, urolithiasis (formation of calculus in the urinary tract), and nephrolithiasis (formation of kidney stones).

Current treatments for gouty arthritis include colchicine, anti-inflammatory drugs, and intraarticular glucocorticoids. The most effective of these, colchicine administered orally, cannot be tolerated by 80 percent of people because of side effects.

Non-absorbable polymers have been used as complexing agents for the treatment of hyperphosphatemia, hypercholesterolemia or urolithiasis:

It has been shown that water soluble polyether glycol polmers are capeable of binding phosphate and oxalate and can therefore be used as an oral composition for treating patients suffering from hyperphosphatemia (WO 98/17707).

Sevelamer hydrochloride, commercially available as RenaGel^{®} (GelTex Pharmaceuticals, Inc., Waltham, MA) is a phosphate-binding gel that is used for clinical control of serum phosphate levels in patients on haemodialysis (Chertow GM et al (1999), Nephrology Dialysis Transplantation, vol. 14, p. 2907-2914).

Colesevelam hydrochloride has been shown to bind bile salts and therefore, can be useful for lowering chloesterol levels (Jones R (2000), Current Opinion in Cardiovascular, Pulmonary and Renal Investigational Drugs, vol. 2, p. 284-291).

Parenterally applied polymeric and copolymeric alkane carboxylic acids with a molecular weight of above 5,000 have been proven to be suitable for controlling urolithiasis (EP 0 303 296).

US 6,177,478 discloses the use of a therapeuticfally effective amount of non-absorbable amine polymers to treat kidney stones by reducing oxalate levels in a patient.

The invention relates to a treatment for gout in an individual with a polymer structurally defined in the claims that binds to uric acid or to a precursor thereof, such as purine. The invention is effective for both treatment and prevention of gout, hyperuricosuria, uric acid nephropathy. Polymers that bind uric acid may also have utility in lowering uric acid levels in a patient at risk of developing coronary heart disease.

The invention relates to the discovery that the polymer, sevelamer hydrochloride (sevelamer hydrogen chloride) can be used to reduce uric acid levels in a patient in need thereof. The invention includes all polymers as defined in the claims which bind to uric acid, or to a precursor thereof, reducing its concentration in plasma. Polymers useful in an embodiment of the invention are polymers containing known uric acid binding agents.

Many functional groups are known uric acid binding agents. These functional groups can be attached to a polymer backbone, preferably a polymer backbone of sufficient molecular weight or of a sufficient degree of cross-linking to prevent or to minimize absorption. Typical substituent groups which can reduce uric acid include amines, cyano groups, acetylene and other olefins, phosphines, arsines, sulfides, dithiocarbamates, nitrates, carboxylates (for example, EDTA, tartrate and oxalate), phenolates, acetylacetonates, and hydroxy groups (for example, glyceryl).

An especially preferred polymer is a cross-linked polyamine. The cross-linking avoids or minimizes absorption of the polymer in the patient. Such polyamines can include polyallylamine, polyethyleneimine (linear or branched), polyvinylamine, polybutenylamine, polylysine, polyarginine, and poly(aminopropylacrylamide). The polyamines can also be substituted with groups which promote binding to uric acid as described above.

Preferred polymers employed in the invention comprise water-insoluble, non-absorbable, and optionally cross-linked polyamines as described herein. The polyamines of the invention can be amine or ammonium-containing aliphatic polymers. An aliphatic amine polymer is a polymer which is manufactured by polymerizing an aliphatic amine monomer. In a preferred embodiment, the polymers are characterized by one or more monomeric units of Formula I: 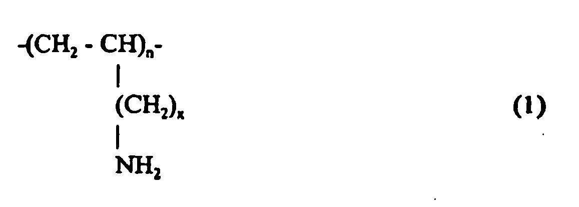 and salts thereof, where n is a positive integer and x is 0 or an integer between 1 and 4, preferably 1. In preferred embodiments, the polymer is cross-linked by means of a multifunctional cross-linking agent.

The invention provides for the use of the polymers described herein for the manufacture of a medicament for the treatment of gout, the medicament "binding" uric acid or urate.

Other features and advantages will be apparent from the following description of the preferred embodiments thereof and from the claims.

As described above, the preferred polymers employed in the invention comprise water-insoluble, non-absorbable, optionally cross-linked polyamines. Preferred polymers are aliphatic. Examples of preferred polymers include polyethylenamine, polyallylamine, polyvinylamine and polydiallylamine polymers. The polymers can be homopolymers or copolymers, as discussed below, and can be substituted or unsubstituted. These and other polymers which can be used in the claimed invention have been reported in United States Patents Nos. 5,487,888; 5,496,545; 5,607,669; 5,618,530; 5,624,963; 5,667,775; 5,679,717; 5,703,188; 5,702,696 and 5,693,675. Polymers suitable for use in the invention are also reported in copending U.S. Applications Serial Nos. 08/659,264; 08/823,699; 08/835,857; 08/470,940; 08/826,197; 08/777,408; 08/927,247; 08/964,498; 08/964,536 and 09/359,226.

The polymer can be a homopolymer or a copolymer of one or more amine-containing monomers or a copolymer of one or more amine-containing monomers in combination with one or more non-amine containing monomers. Where copolymers are manufactured with the monomer of the above Formula I, the comonomers are preferably inert, non-toxic and possess uric acid-binding properties. Examples of suitable non-amine-containing monomers include vinylalcohol, acrylic acid, acrylamide, and vinylformamide. Examples of amine-containing monomers preferably include monomers having the Formula 1 above. Preferably, the monomers are aliphatic. Most preferably, the polymer is a homopolymer, such as a homopolyallylamine, homopolyvinylamine, homopolydiallylamine or polyethylenamine. The word "amine," as used herein, includes primary, secondary and tertiary amines, as well as ammoniums such as trialkylammonium.

The polymers are polymers characterized by one or more repeat units set forth below. 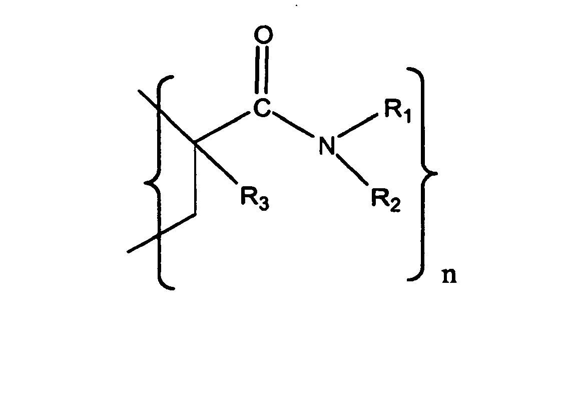 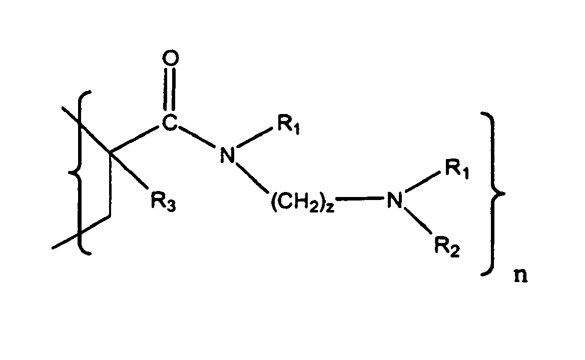 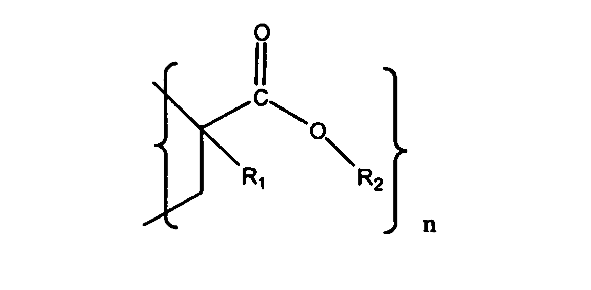 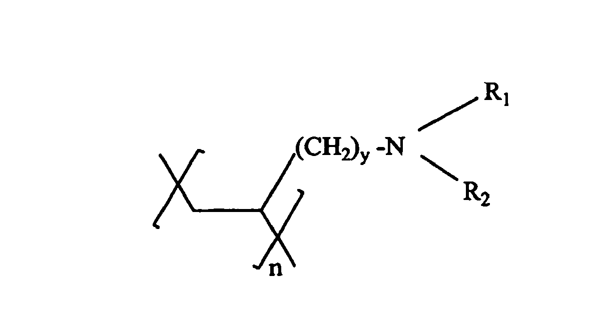 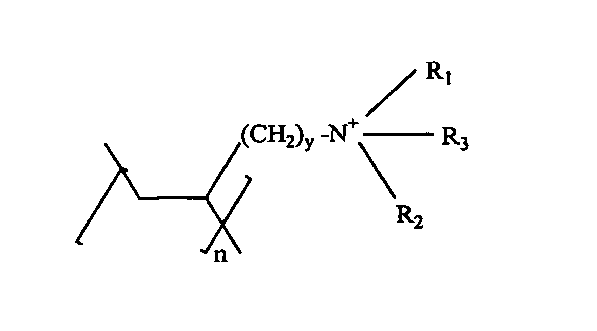 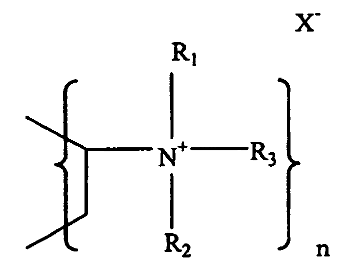 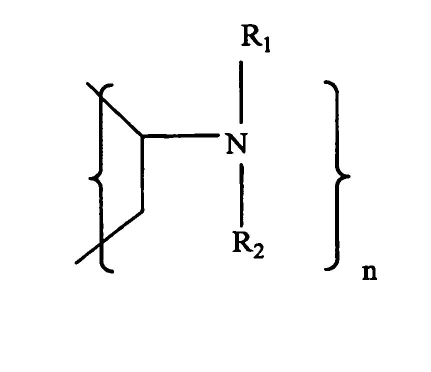 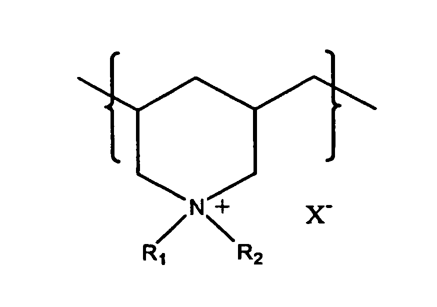 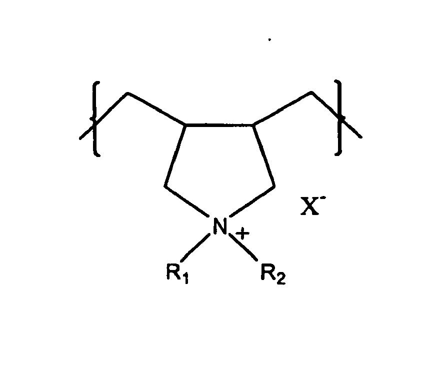 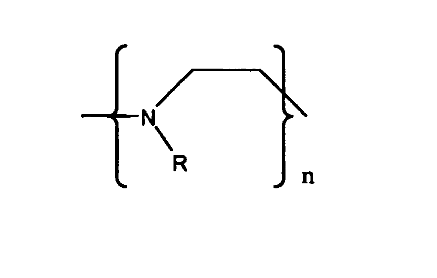 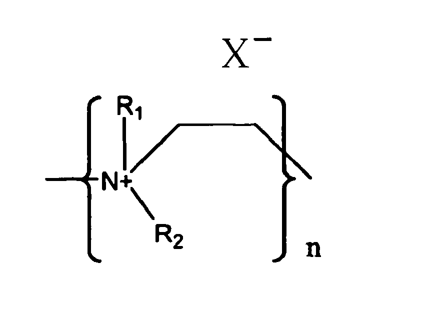 or copolymers thereof, wherein n is a positive integer, y and z are both integers of one or more (e.g., between one and 10) and each R, R₁, R₂, and R₃, independently, is H or a substituted or unsubstituted alkyl group (e.g., having between 1 and 25 or between 1 and 5 carbon atoms, inclusive), alkylamino, (e.g., having between 1 and 5 carbons atoms, inclusive, such as ethylamino or poly(ethylamino)) or aryl (e.g., phenyl) group, and each X' is an exchangeable negatively charged counterion.

In one preferred polymer, at least one of R, R₁, R₂, or R₃ groups is a hydrogen atom. In a more preferred embodiment, each of these groups are hydrogen.

In each case, the R groups can carry one or more substituents. Suitable substituents include therapeutic anionic groups, e.g., quaternary ammonium groups, or amine groups, e.g., primary, secondary or tertiary alkyl or aryl amines. Examples of other suitable substituents include hydroxy, alkoxy, carboxamide, sulfonamide, halogen, alkyl, aryl, hydrazine, guanadine, urea, poly(alkyleneimine), such as poly(ethyleneimine), and carboxylic acid esters.

Preferably, the polymer is rendered water-insoluble by cross-linking. The cross-linking agent can be characterized by functional groups which react with the amino group of the monomer. Alternatively, the cross-linking group can be characterized by two ore more vinyl groups which undergo free radical polymerization with the amine monomer.

Examples of suitable cross-linking agents include diacrylates and dimethylacrylates (e.g. ethylene glycol diacrylate, propylene glycol diacrylate, butylene glycol diacrylate, ethylene glycol dimethacrylate, propylene glycol dimethacrylate, butylene glycol dimethacrylate, polyethyleneglycol dimethacrylate and polyethyleneglycol diacrylate), methylene bisacrylamide, methylene bismethacrylamide, ethylene bisacrylamide, ethylene bismethacrylamide, ethylidene bisacrylamide, divinylbenzene, bisphenol A, dimethacrylate and bisphenol A diacrylate. The cross-linking agent can also include acryloyl chloride, epichlorohydrin, butanediol diglycidyl ether, ethanediol diglycidyl ether, succinyl dichloride, the diglycidal ether of bisphenol A, pyromellitic dianhydride, toluene diisocyanate, ethylene diamine and dimethyl succinate.

Preferably the polymer is non-absorbable in the gastrointestinal tract and/or substantially water-insoluble. The polymer can be characterized by 10 or more monomeric units and/or possess a molecular weight of about 570 or more, preferably about 5,000 daltons or more.

The terms "insoluble," "substantially water-insoluble," and grammatical variations thereof, as used herein, refer to a polymer or other substance which does not dissolve in an aqueous-based system, or which dissolves or solubilizes at a slower rate than does a water-soluble substance. Water-insoluble polymers introduced into the gastrointestinal tract are not absorbed systemically, or are absorbed to a lesser extent than are water-soluble polymers.

"Nonabsorbent" or "non-absorbable," as the terms are used herein, means that the polymer or other substance so described does not dissolve in the gastrointestinal tract, or dissolves to a lesser extent than does an absorbent or absorbable substance, or does not erode, degrade, or otherwise break down *in vitro* to form smaller chemical species by either physical or chemical processes. Therefore, a non-absorbable polymer is not absorbed systemically or is absorbed to a lesser extent than is an absorbable polymer.

A preferred cross-linking agent is epichlorohydrin because of its high availability and low cost. Epichlorohydrin is also advantageous because of its low molecular weight and hydrophilic nature, increasing the water-swellability and gel properties of the polyamine.

The level of cross-linking makes the polymers insoluble and substantially resistant to absorption and degradation, thereby limiting the activity of the polymer to the gastrointestinal tract, and reducing potential side-effects in the patient. The compositions thus tend to be non-systemic in activity. Typically, the cross-linking agent is present in an amount from 0.5-35% or 0.5-25% (such as from 2.5-20% or 1-10%) by weight, based upon total weight of monomer plus cross-linking agent. The polymers can also be further derivatized; examples include alkylated amine polymers, as described, for example, in United States Patent Nos. 5,679,717, 5,607,669 and 5,618,530. Preferred alkylating agents include hydrophobic groups (such as aliphatic hydrophobic groups) and/or quaternary ammonium- or amine-substituted alkyl groups.

Non-cross-linked and cross-linked polyallylamine and polyvinylamine are generally known in the art and are commercially available. Methods for the manufacture of polyallylamine and polyvinylamine, and cross-linked derivatives thereof, are described in the above US Patents. Harada et al. (US Patent Nos. 4,605,701 and 4,528,347), also describe methods of manufacturing polyallylamine and cross-linked polyallylamine.

In other embodiments, the polymer can be a homopolymer or copolymer of polybutenylamine, polylysine, or polyarginine. Alternatively, the polymer can be an aromatic polymer, such as an amine or ammonium-substituted polystyrene, (e.g., cholestyramine).

As described above the polymer can be administered in the form of a salt. By "salt" it is meant that the nitrogen group in the repeat unit is protonated to create a positively charged nitrogen atom associated with a negatively charged counterion. A preferred polymer is a low salt, such as low chloride, form of polyallylamine where less than 40% of the amine groups are protonated.

The cationic counterions can be selected to minimize adverse effects on the patient, as is more particularly described below. Examples of suitable counterions include organic ions, inorganic ions, or a combination thereof, such as halides (Cl⁻ and Br⁻) CH₃OSO₃⁻, HSO₄⁻, SO₄²⁻, HCO₃⁻, CO₃⁻, acetate, lactate, succinate, propionate, oxalate, butyrate, ascorbate, citrate, dihydrogen citrate, tartrate, taurocholate, glycocholate, cholate, hydrogen citrate, maleate, benzoate, folate, an amino acid derivative, a nucleotide, a lipid, or a phospholipid. The counterions can be the same as, or different from, each other. For example, the polymer can contain two different types of counterions.

The polymers, according to an embodiment of the invention, are administered to a patient in a therapeutically effective amount. As used herein, the terms "therapeutically effective amount" and "therapeutically effective dose" refer to the amount of an active agent, for example, a therapeutically effective substance, such as a polymer described herein, required to be administered in order to induce a desired result in the patient. That result may be alleviation or amelioration (complete or partial) of the symptoms or condition of the patient in need of treatment, or any other desired improvement in the patient's symptoms, disease or condition.

As used herein, the term "therapeutically effective amount" may also refer to the quantity of active agent or therapeutically effective substance, such as an amine polymer described herein, the administration of which results in improvement in the patient's symptoms, disease, or condition, where little or no improvement would occur in the absence of the active agent. Typically, the polymer is administered for a sufficient period of time to achieve the desired therapeutic effect.

Therapeutic efficacy may be determined by using standard pharmacological procedures in experimental animals.

The polymers according to the invention can be administered orally to a patient in a dosage comprising between 1 µg/kg/day and 1 g/kg/day. The particular dosage will depend on the individual patient (e.g., the patient's weight and the extent of uric acid removal required) and on the nature of the polymer used. Polymers according to the invention can be administered in one or several doses per day. In one embodiment, it is presently contemplated that, for therapeutic treatments, at least one polymer of the present invention can be administered to an adult in an amount comprising between 70 µg and 91g per day; between 0.1 g and 10 g per day; between 0.5 g and 6 g per day; or between 0.5 g and 3 g per day.

In another embodiment of the invention, the polymers according to the invention can be administered orally to a patient in a dosage of between 1 mg/kg/day and 1 g/kg/day; and between 40 mg/kg/day and 200 mg/kg/day, preferably between 10 mg/kg/day and 200 mg/kg/day. The particular dosage will depend on the individual patient (e.g., the patient's weight and the extent of uric acid removal required). According to an embodiment, the polymer is administrated either in hydrated or dehydrated form. The polymer can be flavored or added to a food or drink, if desired to enhance patient acceptability.

In one embodiment of the invention used to treat chronic gouty arthritis, uric acid nephropathy, or stone disease, the polymer should be administered as soon as possible after an attack.

Uric acid is naturally synthesized by xanthine oxidase-catalyzed oxidation of hypoxanthine and xanthine. Accordingly, in one embodiment, the polymer is administered with a uric acid synthesis inhibitor such as a xanthine oxidase inhibitor. In a particular embodiment, the xanthine oxidase inhibitor includes allopurinol. In another embodiment, the polymer is administered with a uricosuric agent. Uricosuric agents act directly on the renal tubules to increase excretion of uric acid.

In yet another embodiment, additional ingredients, for example, nonsteroidal anti-inflammatory drugs such as colchicine, ingredients for treating other related indications, or inert substances such as artificial coloring agents are added.

According to an embodiment, the additional active ingredients can be administered simultaneously or sequentially with the uric acid-binding polymer. In one embodiment, where the ingredients are administered simultaneously, they can optionally be bound to the polymer, for example, by covalent bonding or a hydrolyzable bonding, or by physically encapsulating the ingredient, on the exterior or interior of the polymeric particle. Covalent bonding can be accomplished by reacting the polymer and ingredient(s) with suitable cross-linking agents.

Examples of suitable forms for administration (preferably oral administration) include pills, tablets, capsules, and powders (e.g., for sprinkling on food or incorporating into a drink). In one embodiment, the pill, tablet, capsule, or powder can be coated with a substance capable of protecting the composition from disintegration in the esophagus but that will allow disintegration as the composition enters the stomach, mixes with food, and passes into the patient's small intestine. The polymer can be administered alone or in combination with a pharmaceutically acceptable carrier substance, e.g., zinc salts, magnesium carbonate, lactose, or a phospholipid with which the polymer can form a micelle.

The polymers of the invention can be used to treat patients, preferably humans, with gout, or as a prophylactic.

### EXEMPLIFICATION

### A. Polymer Preparation

### Example 1 - Poly(vinylamine)

The first step involved the preparation of ethylidenebisacetamide. Acetamide (118 g), acetaldehyde (44.06 g), copper acetate (0.2 g), and water (300 mL) were placed in a 1 L three neck flask fitted with condenser, thermometer, and mechanically stirred. Concentrated HCl (34 mL) was added and the mixture was heated to 45-50°C with stirring for 24 hours. The water was then removed *in vacuo* to leave a thick sludge which formed crystals on cooling to 5°C. Acetone (200 mL) was added and stirred for a few minutes, after which the solid was filtered off and discarded. The acetone was cooled to 0°C and solid was filtered off. The solid was rinsed in 500 mL acetone and air dried 18 hours to yield 31.5 g of ethylidenebisacetamide.

The next step involved the preparation of vinylacetamide from ethylidenebisacetamide. Ethylidenebisacetamide (31.05 g), calcium carbonate (2 g) and filter agent, Celite^{®} 541 (2 g) (available from Aldrich, Milwaukee, WI) were placed in a 500 mL three neck flask fitted with a thermometer, a mechanical stirrer, and a distilling head atop a Vigreaux column. The mixture was vacuum distilled at 24 mm Hg by heating the pot to 180-225°C. Only a single fraction was collected (10.8 g) which contained a large portion of acetamide in addition to the product (determined by NMR). This solid product was dissolved in isopropanol (30 mL) to form the crude vinylacetamide solution used for polymerization.

Crude vinylacetamide solution (15 mL), divinylbenzene (1 g, technical grade, 55% pure, mixed isomers), and AIBN (0.3 g) were mixed and heated to reflux under a nitrogen atmosphere for 90 minutes, forming a solid precipitate. The solution was cooled, isopropanol (50 mL) was added, and the solid was collected by centrifugation. The solid was rinsed twice in isopropanol, once in water, and dried in a vacuum oven to yield 0.8 g of poly(vinylacetamide), which was used to prepare poly(vinylamine) as follows.

Poly(vinylacetamide) (0.79 g) was placed in a 100 mL one neck flask containing water (25 mL) and conc. HCl (25 mL). The mixture was refluxed for 5 days, after which the solid was filtered off, rinsed once in water, twice in isopropanol, and dried in a vacuum oven to yield 0.77 g of product. Infrared spectroscopy indicated that a significant amount of the amide (1656 cm⁻¹) remained and that not much amine (1606 cm⁻¹) was formed. The product of this reaction (~0.84 g) was suspended in NaOH (46 g) and water (46 g) and heated to boiling (~140°C). Due to foaming the temperature was reduced and maintained at ~100°C for 2 hours. Water (100 mL) was added and the solid collected by filtration. After rinsing once in water the solid was suspended in water (500 mL) and adjusted to pH 5 with acetic acid. The solid was again filtered off, rinsed with water, then isopropanol, and dried in a vacuum oven to yield 0.51 g of product. Infrared spectroscopy indicated that significant amine had been formed.

### Example 2 - Poly(allylamine) hydrochloride

To a 2 liter, water-jacketed reaction kettle equipped with (1) a condenser topped with a nitrogen gas inlet, (2) a thermometer, and (3) a mechanical stirrer was added concentrated hydrochloric acid (360 mL). The acid was cooled to 5°C using circulating water in the jacket of the reaction kettle (water temperature = 0°C). Allylamine (328.5 mL, 250 g) was added dropwise with stirring while maintaining the reaction temperature at 5-10°C. After addition was complete, the mixture was removed, placed in a 3 liter one-neck flask, and 206 g of liquid was removed by rotary vacuum evaporation at 60°C. Water (20 mL) was then added and the liquid was returned to the reaction kettle. Azobis(amidinopropane) dihydrochloride (0.5 g) was suspended in 11 mL of water was then added. The resulting reaction mixture was heated to 50°C under a nitrogen atmosphere with stirring for 24 hours. Additional azobis(amidinopropane) dihydrochloride (5 mL) suspended in 11 mL of water was then added, after which heating and stirring were continued for an additional 44 hours.

At the end of this period, distilled water (100 mL) was added to the reaction mixture and the liquid mixture allowed to cool with stirring. The mixture was then removed and placed in a 2 liter separatory funnel, after which it was added dropwise to a stirring solution of methanol (4 L), causing a solid to form. The solid was removed by filtration, re-suspended in methanol (4 L), stirred for 1 hour, and collected by filtration. The methanol rinse was then repeated one more time and the solid dried in a vacuum oven to afford 215.1 g of poly(allylamine) hydrochloride as a granular white solid.

### Example 3 - Poly(allylamine) hydrochloride cross-linked with epichlorohydrin

To a 5 gallon vessel was added poly(allylamine) hydrochloride prepared as described in Example 2(1 kg) and water (4 L). The mixture was stirred to dissolve the hydrochloride and the pH was adjusted by adding solid NaOH (284 g). The resulting solution was cooled to room temperature, after which epichlorohydrin cross-linking agent (50 mL) was added all at once with stirring. The resulting mixture was stirred gently until it gelled (about 35 minutes). The cross-linking reaction was allowed to proceed for an additional 18 hours at room temperature, after which the polymer gel was removed and placed in portions in a blender with a total of 10 L of water. Each portion was blended gently for about 3 minutes to form coarse particles which were then stirred for 1 hour and collected by filtration. The solid was rinsed three times by suspending it in water (10 L, 15 L, 20 L), stirring each suspension for 1 hour, and collecting the solid each time by filtration. The resulting solid was then rinsed once by suspending it in isopropanol (17 L), stirring the mixture for 1 hour, and then collecting the solid by filtration, after which the solid was dried in a vacuum oven at 50°C for 18 hours to yield about 677 g of the cross-linked polymer as a granular, brittle, white solid.

### Example 4 - Poly(allylamine) hydrochloride cross-linked with butanediol diglycidyl ether

To a 5 gallon plastic bucket was added poly(allylamine) hydrochloride prepared as described in Example 2 (500 g) and water (2 L). The mixture was stirred to dissolve the hydrochloride and the pH was adjusted to 10 by adding solid NaOH (134.6 g). The resulting solution was cooled to room temperature in the bucket, after which 1,4-butanediol diglycidyl ether cross-linking agent (65 mL) was added all at once with stirring. The resulting mixture was stirred gently until it gelled (about 6 minutes). The cross-linking reaction was allowed to proceed for an additional 18 hours at room temperature, after which the polymer gel was removed and dried in a vacuum oven at 75°C for 24 hours. The dry solid was then ground and sieved to -30 mesh, after which it was suspended in 6 gallons of water and stirred for 1 hour. The solid was then filtered off and the rinse process repeated two more times. The resulting solid was then air dried for 48 hours, followed by drying in a vacuum oven at 50°C for 24 hours to yield about 415 g of the cross-linked polymer as a white solid.

### Example 5 - Poly(allylamine) hydrochloride cross-linked with ethanediol diglycidyl ether

To a 100 mL beaker was added poly(allylamine) hydrochloride prepared as described in Example 2 (10 g) and water (40 mL). The mixture was stirred to dissolve the hydrochloride and the pH was adjusted to 10 by adding solid NaOH. The resulting solution was cooled to room temperature in the beaker, after which 1,2-ethanediol diglycidyl ether cross-linking agent (2.0 mL) was added all at once with stirring. The resulting mixture was stirred gently until it gelled (about 4 minutes). The cross-linking reaction was allowed to proceed for an additional 18 hours at room temperature, after which the polymer gel was removed and blended in 500 mL of methanol. The solid was then filtered off and suspended in water (500 mL). After stirring for 1 hour, the solid was filtered off and the rinse process repeated. The resulting solid was rinsed twice in isopropanol (400 mL) and then dried in a vacuum oven at 50°C for 24 hours to yield 8.7 g of the cross-linked polymer as a white solid.

### Example 6 - Poly(allylamine) hydrochloride cross-linked with dimethylsuccinate

To a 500 mL round bottom flask was added poly(allylamine) hydrochloride prepared as described in Example 2 (10 g), methanol (100 mL), and triethylamine (10 mL). The mixture was stirred and dimethylsuccinate cross-linking agent (1 mL) was added. The solution was heated to reflux and the stirring discontinued after 30 minutes. After 18 hours, the solution was cooled to room temperature, and the solid filtered off and blended in 400 mL of isopropanol. The solid was then filtered off and suspended in water (1L). After stirring for 1 hour, the solid was filtered off and the rinse process repeated two more times. The solid was then rinsed once in isopropanol (800 mL) and dried in a vacuum oven at 50°C for 24 hours to yield 5.9 g of the cross-linked polymer as a white solid.

### Example 7 - Poly(allyltrimethylammonium chloride)

To a 500 mL three-necked flask equipped with a magnetic stirrer, a thermometer, and a condenser topped with a nitrogen inlet, was added poly(allylamine) cross-linked with epichlorohydrin (5.0 g), methanol (300 mL), methyl iodide (20 mL), and sodium carbonate (50 g). The mixture was then cooled and water was added to total volume of 2 L. Concentrated hydrochloric acid was added until no further bubbling resulted and the remaining solid was filtered off. The solid was rinsed twice in 10% aqueous NaCl (1 L) by stirring for 1 hour followed by filtration to recover the solid. The solid was then rinsed three times by suspending it in water (2 L), stirring for 1 hour, and filtering to recover the solid. Finally, the solid was rinsed as above in methanol and dried in a vacuum over at 50°C for 18 hours to yield 7.7 g of white granular solid.

### Example 8 - Poly(ethyleneimine)/acryloyl chloride

Into a 5 L three-neck flask equipped with a mechanical stirrer, a thermometer, and an additional funnel was added polyethyleneimine (510 g of a 50% aqueous solution (equivalent to 255 g of dry polymer) and isopropanol (2.5 L). Acryloyl chloride (50 g) was added dropwise through the addition funnel over a 35 minute period, keeping the temperature below 29°C. The solution was then heated to 60°C with stirring for 18 hours. The solution was cooled and solid immediately filtered off. The solid was rinsed three times by suspending it in water (2 gallons), stirring for 1 hour, and filtering to recover the solid. The solid was rinsed once by suspending it in methanol (2 gallons), stirring for 30 minutes, and filtering to recover the solid. Finally, the solid was rinsed as above in isopropanol and dried in a vacuum over at 50°C for 18 hours to yield 206 g of light orange granular solid.

### Example 9 - Poly(dimethylaminopropylacrylamide)

Dimethylamino-propylacrylamide (10g) and methylene-bisacrylamide (1.1 g) were dissolved in 50 mL of water in a 100 mL three-neck flask. The solution was stirred under nitrogen for 10 minutes. Potassium persulfate (0.3 g) and sodium metabisulfite (0.3 g) were each dissolved in 2-3 mL of water and then mixed. After a few seconds this solution was added to the monomer solution, still under nitrogen. A gel formed immediately and was allowed to sit overnight. The gel was removed and blended with 500 mL of isopropanol. The solid was filtered off and rinsed three times with acetone. The solid white powder was filtered off and dried in a vacuum oven to yield 6.1 g.

### Example 10 - Poly(Methacrylamidopropyltrimethylammoniumchloride) = (Poly (MAPTAC))

(3-(Methacryloylamino)propyl)trimethylammonium chloride (38 mL of 50% aqueous solution) and methylenebis-methacrylamide (2.2 g) were stirred in a beaker at room temperature. Methanol (10 mL) was added and the solution was warmed to 40°C to fully dissolve the bisacrylamide. Potassium persulfate (0.4 g) was added and the solution stirred for 2 minutes. Potassium metabisulfite (0.4 g) was added and stirring was continued. After 5 minutes the solution was put under a nitrogen atmosphere. After 20 minutes the solution contained significant precipitate and the solution was allowed to sit overnight. The solid was washed three times with isopropanol and collected by filtration. The solid was then suspended in water 500 (mL) and stirred for several hours before being collected by centrifugation. The solid was again washed with water and collected by filtration. The solid was then dried in a vacuum oven to yield 21.96 g.

### Example 11 - Poly(ethyleneimine) "A"

Polyethyleneimine (50 g of a 50% aqueous solution; Scientific Polymer Products) was dissolved in water (100 mL). Epichlorohydrin (4.6 mL) was added dropwise. The solution was heated to 55°C for 4 hours, after which it had gelled. The gel was removed, blended with water (1 L) and the solid was filtered off. It was resuspended in water (2 L) and stirred for 10 minutes. The solid was filtered off, the rinse repeated once with water and twice with isopropanol, and the resulting gel was dried in a vacuum oven to yield 26.3 g of a rubbery solid.

Poly(ethyleneimine) "B" and Poly(ethyleneimine) "C" were made in a similar manner, except using 9.2 and 2.3 mL of epichlorohydrin, respectively.

### Example 12 - Poly(methylmethacrylate-co-divinylbenzene)

Methylmethacrylate (50 g) and divinylbenzene (5 g) and azobisisobutyronitrile (1.0 g) were dissolved in isopropanol (500 mL) and heated to reflux for 18 hours under a nitrogen 14 atmosphere. The solid white precipitate was filtered off, rinsed once in acetone (collected by centrifugation), once in water (collected by filtration) and dried in a vacuum oven to yield 19.4 g.

### Example 13 - Poly(diethylenetriaminemethacrylamide)

Poly(methyl-methacrylate-co-divinylbenzene) (20 g) was suspended in diethylenetriamine (200 mL) and heated to reflux under a nitrogen atmosphere for 18 hours. The solid was collected by filtration, resuspended in water (500 mL), stirred 30 minutes, filtered off, resuspended in water (500 mL), stirred 30 minutes, filtered off, rinsed briefly in isopropanol, and dried in a vacuum oven to yield 18.0 g.

Poly(pentaethylenehexaminemethacrylamide), Poly(tetraethylenepentaminemethacrylamide), and Poly(triethylenetetraaminemethacrylamide) were made in a manner similar to poly(diethylenetriaminemethacrylamide) from pentaethylenehexamine, tetraethylenepentamine, and triethylenetetraamine, respectively.

### Example 14 - Poly(methylmethacrylate/PEI)

Poly(methylmethacrylate-co-divinylbenzene) (1.0 g) was added to a mixture containing hexanol (9150 mL) and polyethyleneimine (15 g in 15 g water). The mixture was heated to reflux under nitrogen for 4 days. The reaction was cooled and the solid was filtered off, suspended in methanol (300 mL), stirred 1 hour, and filtered off. The rinse was repeated once with isopropanol and the solid was dried in a vacuum oven to yield 0.71 g.

### Example 15 - Poly(aminoethylmethacrylamide)

Poly(methylmethacrylate-co-divinylbenzene) (20 g) was suspended in ethylenediamine 9200 mL) and heated to reflux under a nitrogen atmosphere for 3 days. The solid was collected by centrifugation, washed by resuspending it in water (500 mL), stirring for 30 minutes, and filtering off the solid. The solid was washed twice more in water, once in isopropanol, and dried in a vacuum oven to yield 17.3 g.

### Example 16 - Poly(diethylaminopropylmethacrylamide)

Poly(methyl-methacrylate-co-divinylbenzene) (20 g) was suspended in diethylaminopropylamine (200 mL) and heated to reflux under a nitrogen atmosphere for 18 hours. The solid was collected by filtration, resuspended in water (500 mL), filtered off, resuspended in water (500 mL), collected by filtration, rinsed briefly in isopropanol, and dried in a vacuum oven to yield 8.2 g.

### Example 17 - NHS-acrylate

N-Hydroxysuccinimide (NHS, 157.5 g) was dissolved in chloroform (2300 mL) in a 5 L flask. The solution was cooled to 0°C and acryloyl chloride (132 g) was added dropwise, keeping the temperature at 2°C. After addition was complete, the solution was stirred for 1.5 hours, rinsed with water (1100 mL) in a separatory funnel and dried over anhydrous sodium sulfate. The solvent was removed under vacuum, and a small amount of ethyl acetate was added to the residue. This mixture was poured into hexane (200 mL) with stirring. The solution was heated to reflux, adding more ethyl acetate (400 mL). The insoluble NHS was filtered off, hexane (1 L) was added, the solution was heated to reflux, ethyl acetate (400 mL) was added, and the solution allowed to cool to <10°C. The solid was then filtered off and dried in a vacuum oven to yield 125.9 g. A second crop of 80 g was subsequently collected by further cooling.

### Example 18 - Poly(NHS-acrylate)

NHS-acrylate (28.5 g), methylenebis-acrylamide (1.5 g) and tetrahydrofuran (500 mL) were mixed in a 1 L flask and heated to 50°C under a nitrogen atmosphere. Azobisisobutyronitrile (0.2 g) was added, the solution was stirred for 1 hour, filtered to remove excess N-hydroxysuccinimide, and heated to 50°C for 4.5 hours under a nitrogen atmosphere. The solution was then cooled and the solid was filtered off, rinsed in tetrahydrofuran, and dried in a vacuum oven to yield 16.1 g.

### Example 19 - Poly(guanidinobutylacrylamide)

Poly(NHS-acrylate) (1.5 g) was suspended in water (25 mL) containing agmatine (1.5 g) which had been adjusted to pH 9 with solid NaOH. The solution was stirred for 4 days, after which time the pH had dropped to 6.3. Water was added to a total of 500 mL, the solution was stirred for 30 minutes and the solid was filtered off. The solid was rinsed twice in water, twice in isopropanol, and dried in a vacuum oven to yield 0.45 g.

### Example 20 - Poly(methacryloyl chloride)

Methacryloyl chloride (20 mL), divinyl benzene (4 mL of 80% purity), AIBN (0.4 g), and THF (150 mL) were stirred at 60°C under a nitrogen atmosphere for 18 hours. The solution was cooled and the solid was filtered off, rinsed in THF, then acetone, and dried in a vacuum oven to yield 8.1 g.

### Example 21 - Poly(guanidinobutylmethacrylamide)

Poly(methacryloyl chloride) (0.5 g), agmatine sulfate (1.0 g), triethylamine (2.5 mL), and acetone (50 mL) were stirred together for 4 days. Water (100 mL) was added and the mixture stirred for 6 hours. The solid was filtered off and washed by resuspending in water (500 mL), stirring for 30 minutes, and filtering off the solid. The wash was repeated twice in water, once in methanol, and the solid was dried in a vacuum oven to yield 0.41 g.

### Example 22 - Poly(guanidinoacrylamide)

The procedure for poly-(guanidinobutylacrylamide) was followed substituting aminoguanidine bicarbonate (5.0 g) for the agmatine sulfate, yielding 0.75 g.

### Example 23 - Poly(PEH/EPI)

Epichlorohydrin (1.5 g) was added dropwise to a solution containing pentaethylenehexamine (PEH) (20 g) and water (100 mL), keeping the temperature at about 65°C. The solution was stirred until it gelled and heating was continued for 4 hours (at 65°C). After sitting overnight at room temperature the gel was removed and blended with water (1 L). The solid was filtered off, water was added (1 L), and the blending and filtration were repeated. The gel was suspended in isopropanol and the resulting solid was collected by filtration and dried in a vacuum oven to yield 28.2 g.

### Example 24 - Ethylidenebisacetamide

Acetamide (118 g), acetaldehyde (44.06 g), copper acetate (0.2 g), and water (300 mL) were placed in a 1 L three-neck flask fitted with condenser, thermometer, and mechanical stirred. Concentrated HCl (34 mL) was added and the mixture was heated to 45-50°C with stirring for 24 hours. The water was then removed in vacuo to leave a thick sludge which formed crystals on cooling to 5°C. Acetone (200 mL) was added and stirred for a few minutes after which the solid was filtered off and discarded. The acetone was cooled to 0°C and solid was filtered off. This solid was rinsed in 500 mL acetone and air dried 18 hours to yield 31.5 g.

### Example 25 - Vinylacetamide

Ethylidenebisacetamide (31.05), calcium carbonate (2 g) and Celite 541^{®} (2 g) were placed in a 500 mL three-neck flask fitted with a thermometer, a mechanical stirrer, and a distilling head atop a Vigreaux column. The mixture was vacuum distilled at 35 mm Hg by heating the pot to 180-225°C. Only a single fraction was collected (10.8 g) which contained a large portion of acetamide in addition to the product (determined by NMR). This solid product was dissolved in isopropanol (30 mL) to form the crude solution used for polymerization.

### Example 26 - Poly(vinylacetamide)

Crude vinylacetamide solution (15 mL), divinylbenzene (1 g, technical grade, 55% pure, mixed isomers), and AIBN (0.3 g) were mixed and heated to reflux under a nitrogen atmosphere for 90 minutes, forming a solid precipitate. The solution was cooled, isopropanol (50 mL) was added, and the solid was collected by centrifugation. The solid was rinsed twice in isopropanol, once in water, and dried in a vacuum oven to yield 0.8 g.

### Example 27 - Poly(vinylamine)

Poly(vinylacetamide) (0.79 g) was placed in a 100 mL one neck flask containing water 25 mL and concentrated HCl 25 mL. The mixture was refluxed for 5 days, the solid was filtered off, rinsed once in water, twice in isopropanol, and dried in a vacuum oven to yield 0.77 g. The product of this reaction (~0.84 g) was suspended in NaOH (46 g) and water (46 g) and heated to boiling (~140°C). Due to foaming, the temperature was reduced and maintained at ~100°C for 2 hours. Water (100 mL) was added and the solid collected by filtration. After rinsing once in water, the solid was suspended in water (500 mL) and adjusted to pH 5 with acetic acid. The solid was again filtered off, rinsed with water, then the isopropanol, and dried in a vacuum oven to yield 0.51 g.

### Example 28 - Poly(ethyleneimine) Salts

Polyethyleneimine (25 g dissolved in 25 g water) was dissolved in water (100 mL) and mixed with toluene (1 L). Epichlorohydrin (2.3 mL) was added and the mixture heated to 60°C with vigorous mechanical stirring for 18 hours. The mixture was cooled and the solid filtered off, resuspended in methanol (2 L), stirred 1 hour, and collected by centrifugation. The solid was suspended in water (2 L), stirred 1 hour, filtered off, suspended in water (4 L), stirred 1 hour, and again filtered off. The solid was suspended in acetone (4 L) and stirred 15 minutes, the liquid was poured off, acetone (2 L) was added, the mixture was stirred 15 minutes, the acetone was again poured off, and the solid was dried in a vacuum oven to form intermediate "D".

### Example 29 - Poly(ethyleneimine sulfate A)

Intermediate "D" (1.0 g) was suspended in water (150 mL), stirred 30 minutes, and partially neutralized with sulfuric acid (1.1 g). The mixture was stirred an additional 30 minutes, the solid was filtered off, resuspended in methanol (200 mL), stirred 5 minutes, filtered off, and dried in a vacuum oven.

### Example 30 - Poly(ethyleneimine sulfate B)

Intermediate "D" (1.0 g) was suspended in water (150 mL), stirred 30 minutes, and partially neutralized with sulfuric acid (0.57 g). The mixture was stirred an additional 30 minutes, the solid was filtered off, resuspended in methanol (200 mL), stirred 5 minutes, filtered off, and dried in a vacuum oven.

### Example 31 - Poly(ethyleneimine sulfate C)

Intermediate "D" (1.0 g) was suspended in water (150 mL), stirred 30 minutes, and partially neutralized with sulfuric acid (0.28 g). The mixture was stirred an additional 30 minutes, the solid was filtered off, resuspended in methanol (200 mL), stirred 5 minutes, filtered off, and dried in a vacuum oven.

### Example 32 - Poly(ethyleneimine sulfate D)

Intermediate "D" (1.0 g) was suspended in water (150 mL), stirred 30 minutes, and partially neutralized with sulfuric acid (0.11 g). The mixture was stirred an additional 30 minutes, the solid was filtered off, resuspended in methanol (200 mL), stirred 5 minutes, filtered off, and dried in a vacuum oven.

### Example 33 - Poly(ethyleneimine tartrate A)

Intermediate "D" (1.0 g) was suspended in water (150 mL), stirred 30 minutes, and partially neutralized with tartaric acid (1.72 g). The mixture was stirred an additional 30 minutes, the solid was filtered off, resuspended in methanol (200 mL), stirred 5 minutes, filtered off, and dried in a vacuum oven.

### Example 34 - Poly(ethyleneimine tartrate B)

Intermediate "D" (1.0 g) was suspended in water (150 mL), stirred 30 minutes, and partially neutralized with tartaric acid (0.86 g). The mixture was stirred an additional 30 minutes, the solid was filtered off, resuspended in methanol (200 mL), stirred 5 minutes, filtered off, and dried in a vacuum oven.

### Example 35 - Poly(ethyleneimine tartrate C)

Intermediate "D" (1.0 g) was suspended in water (150 mL), stirred 30 minutes, and partially neutralized with tartaric acid (0.43 g). The mixture was stirred an additional 30 minutes, the solid was filtered off, resuspended in methanol (200 mL), stirred 5 minutes, filtered off, and dried in a vacuum oven.

### Example 36 - Poly(ethyleneimine ascorbate A)

Intermediate "D" (1.0 g) was suspended in water (150 mL), stirred 30 minutes, and partially neutralized with ascorbic acid (4.05 g). The mixture was stirred an additional 30 minutes, the solid was filtered off, resuspended in methanol (200 mL), stirred 5 minutes, filtered off, and dried in a vacuum oven.

### Example 37 - Poly(ethyleneimine ascorbate B)

Intermediate "D" (1.0 g) was suspended in water (150 mL), stirred 30 minutes, and partially neutralized with ascorbic acid (2.02 g). The mixture was stirred an additional 30 minutes, the solid was filtered off, resuspended in methanol (200 mL), stirred 5 minutes, filtered off, and dried in a vacuum oven.

### Example 38 - Poly(ethyleneimine ascorbate C)

Intermediate "D" (1.0 g) was suspended in water (150 mL), stirred 30 minutes, and partially neutralized with ascorbic acid (1.01 g). The mixture was stirred an additional 30 minutes, the solid was filtered off, resuspended in methanol (200 mL), stirred 5 minutes, filtered off, and dried in a vacuum oven.

### Example 39 - Poly(ethyleneimine citrate A)

Intermediate "D" (1.0 g) was suspended in water (150 mL), stirred 30 minutes, and partially neutralized with citric acid (1.47 g). The mixture was stirred an additional 30 minutes, the solid was filtered off, resuspended in methanol (200 mL), stirred 5 minutes, filtered off, and dried in a vacuum oven.

### Example 40 - Poly(ethyleneimine citrate B)

Intermediate "D" (1.0 g) was suspended in water (150 mL), stirred 30 minutes, and partially neutralized with citric acid (0.74 g). The mixture was stirred an additional 30 minutes, the solid was filtered off, resuspended in methanol (200 mL), stirred 5 minutes, filtered off, and dried in a vacuum oven.

### Example 41 - Poly(ethyleneimine citrate C)

Intermediate "D" (1.0 g) was suspended in water (150 mL), stirred 30 minutes, and partially neutralized with citric acid (0.37 g). The mixture was stirred an additional 30 minutes, the solid was filtered off, resuspended in methanol (200 mL), stirred 5 minutes, filtered off, and dried in a vacuum oven.

### Example 42 - Poly(ethyleneimine succinate A)

Intermediate "D" (1.0 g) was suspended in water (150 mL), stirred 30 minutes, and partially neutralized with succinic acid (1.36 g). The mixture was stirred an additional 30 minutes, the solid was filtered off, resuspended in methanol (200 mL), stirred 5 minutes, filtered off, and dried in a vacuum oven.

### Example 43 - Poly(ethyleneimine succinate B)

Intermediate "D" (1.0 g) was suspended in water (150 mL), stirred 30 minutes, and partially neutralized with succinic acid (0.68 g). The mixture was stirred an additional 30 minutes, the solid was filtered off, resuspended in methanol (200 mL), stirred 5 minutes, filtered off, and dried in a vacuum oven.

### Example 44 - Poly(ethyleneimine chloride)

Polyethyleneimine (100 g in 100 g water) was dissolved in water (640 mL additional) and the pH was adjusted to 10 with concentrated HCl. Isopropanol (1.6 L) was added, followed by epichlorohydrin (19.2 mL). The mixture was stirred under nitrogen for 18 hours at 60°C. The solids were filtered off and rinsed with methanol (300 mL) on the funnel. The solid was rinsed by resuspending it in methanol (4 L), stirring 30 minutes, and filtering off the solid. The rinse was repeated twice with methanol, followed by resuspension in water (1 gallon). The pH was adjusted to 1.0 with concentrated HCl, the solid was filtered off, resuspended in water (1 gallon), the pH again adjusted to 1.0 with concentrated HCl, the mixture stirred 30 minutes, and the solid filtered off. The methanol rinse was again repeated and the solid dried in a vacuum oven to yield 112.4 g.

### Example 45 - Poly(dimethylethyleneimine chloride)

Poly(ethyleneimine chloride) (5.0 g) was suspended in methanol (300 mL) and sodium carbonate (50 g) was added. Methyl iodide (20 mL) was added and the mixture heated to reflux for 3 days. Water was added to reach a total volume of 500 mL, the mixture stirred for 15 minutes, and the solid filtered off The solid was suspended in water (500 mL), stirred 30 minutes, and filtered. The solid was suspended in water (1 L), the pH adjusted to 7.0 with concentrated HCl, and the mixture stirred for 10 minutes. The solid was filtered off, resuspended in isopropanol (1 L), stirred 30 minutes, filtered off, and dried in a vacuum oven to yield 6.33 g.

### Example 46 - Poly(methacryloyl chloride)

Methacryloyl chloride (20 mL), divinyl benzene (4 mL of 80% purity), AIBN (0.4 g), and THF (150 mL) were stirred at 60°C under a nitrogen atmosphere for 18 hours. The solution was cooled, and the solid was filtered off, rinsed in THF, then acetone, and dried in a vacuum oven to yield 8.1 g.

### Example 47 - Poly(guanidinobutylmethacrylamide)

Poly(methacryloyl chloride) (0.5 g), agmatine sulfate (1.0 g), triethylamine (2.5 mL), and acetone (50 mL) were stirred together for 4 days. Water (100 mL) was added, and the mixture stirred for 6 hours. The solid was filtered off, washed by resuspending in water (500 mL), stirring for 30 minutes, and filtering off the solid. The wash was repeated twice in water, once in methanol, and the solid was dried in a vacuum oven to yield 0.41 g.

### Example 48 - Poly(PEH/EPI)

Epichlorohydrin (21.5 g) was added dropwise to a solution containing pentaethylenehexamine (20 g) and water (100 mL), keeping the temperature below 65°C. The solution was stirred until it gelled, and heating was continued for 4 hours (at 65°C). After sitting overnight at room temperature, the gel was removed and blended with water (1 L). The solid was filtered off, water was added (1 L), and the blending and filtration were repeated. The gel was suspended in isopropanol, and the resulting solid was collected by filtration and dried in a vacuum oven to yield 28.2 g.

### Example 49 - Poly(TAEA-acrylamide)

Poly(NHS-acrylate) (4.4 g) was suspended in a solution containing water (100 mL) and tris(2-aminoethyl)amine (30 mL) which had been adjusted to pH 9 with concentrated HCl. After 4 days of stirring, the solid was filtered off, and the wash repeated. The solid was then rinsed briefly with water twice, isopropanol once, and dried in a vacuum oven to yield 3.4 g.

### Example 50 - Poly(PEH-acrylamide)

Poly(NHS-acrylate) (5.0 g) was suspended in a solution containing water (100 mL) and pentaethylene hexamine (30 mL) which had been adjusted to pH 10 with concentrated HCI. After 4 days of stirring, the solid was filtered off and resuspended in water (500 mL). The mixture was stirred for 4 hours, the solid was filtered off, and the wash repeated. The solid was then rinsed briefly with water twice, isopropanol once, and dried in a vacuum oven to yield 4.7 g.

### Example 51 - Poly(MI/EPI)

To a 500 mL flask was added 2-methylimidazole (41.00 g, 0.50 mol) and water (100 mL). The solution was heated to 55°C, and epichlorohydrin (46.3 g. 0.50 mol) was added dropwise over 100 minutes. The maximum temperature reached during the addition was 75°C. When the addition was complete, the solution was heated to 90°C and held at that temperature for 18 hours. In the morning, the reaction was cooled to 45°C, and epichlorohydrin (8.7 g, 0.094 mol) was added dropwise. After the addition was complete, the solution was stirred at 45°C for 2 hours. At this point, a solution of sodium hydroxide (3.78 g, 0.094 mol) in water (15 mL) was prepared. The reaction was cooled, and the sodium hydroxide solution was added dropwise at 28°C over 10 minutes. The solution was stirred for an additional 15 minutes and then transferred to a beaker and heated to 95°C on a hot plate. When the reaction solidified, it was placed in an oven at 125°C for 5 hours to cure. After cooling to room temperature, the polymer was broken up and added to 2000 mL of water. The mixture was allowed to stand for 3 hours and then blended in two portions. The hydrated gel was filtered and then dehydrated with isopropanol in two steps in the blender. Filtration and vacuum drying afforded 83.51 g of title polymer.

### Example 52 - Polyallylamine cross-linked with epichlorohydrin

An aqueous solution of poly(allylamine hydrochloride) (500 lb of a 50.7% aqueous solution) was diluted with water (751 1b) and neutralized with aqueous sodium hydroxide (171 1b of a 50% aqueous solution). The solution was cooled to approximately 25°C, and acetonitrile (1340 lb) and epichlorohydrin (26.2 lb) were added. The solution was stirred vigorously for 21 hours. During this time, the reactor contents changed from two liquid phases to a slurry of particles in a liquid. The solid gel product was isolated by filtration. The gel was washed in an elutriation process with water (136,708 b). The gel was isolated by filtration and rinsed with isopropanol. The gel was slurried with isopropanol (1269 lb) and isolated by filtration. The isopropanol/water wet gel was dried in a vacuum dryer at 60°C. The dried product was ground to pass through a 50 mesh screen to give a product suitable for pharmacologic use (166 lb, 73%).

### B. Clinical Testing

### Haemodialysis Patient Studies

Protocols 1-6 for use of RenaGel^{®} (Geltex Pharmaceuticals, Inc., Waltham, MA) in each of the haemodialysis patient studies are provided in the following references, respectively.

### Protocol 1:

Chertow, G.M., Burke, S.K., Lazarus, J.M.., Stenzel, K.H., Wombolt, D., Goldberg, D., Bonventre, J.V., and Slatopolsky, E., "Poly(allylamine hydrochloride) (RenaGel®): a noncalcemic phosphate binder for the treatment of hyperphosphatemia in chronic renal failure," Am JKid Dis. 29: 66-71 (1997).

### Protocol 2:

Goldberg, D.I., Dillon, M.A., Slatapolsky, E.A., Garrett ,B., Gray, J.R., Marbury, T., Weinberg, M., Wombolt, D., and Burke, S.K., "Effect of RenaGel, a non-absorbed, calcium-and aluminum-free phosphate binder, on serum phosphorus, calcium, and intact parathyroid hormone in end-stage renal disease patients," Nephrol Dial Transplant. 13: 2303-2310 (1998).

### Protocol 3:

Chertow, G.M., Dillon, M., Burke, S.K., Steg, M., Bleyer, A.J., Garrett, B.N., Domoto, D.T., Wilkes, B.M., Wombolt, D.G., and Slatopolsky, E., "A randomized trial of sevelamer hydrochloride (RenaGel®) with and without supplemental calcium. Strategies for the control of hyperphosphatemia in hemodialysis patients," Clin Nephrol. 51: 18-26 (1999).

### Protocol 4:

Bleyer, A.J., Burke, S.K., Dillon, M., Garrett, B., Kant, K.S., Lynch, D., Raman, S.N., Shoenfeld, P., Teitelbaum, I., Zieg, S., and Slatopolsky, E., "A comparison of the calcium-free phosphate binder sevelamer hydrochloride with calcium acetate in the treatment of hyperphosphatemia in hemodialysis patients," Am JKid Dis. 33: 694-701 (1999).

### Protocol 5:

Slatopolsky, E., Burke, S.K., Dillon, M.A., and the RenaGel Study Group, "RenaGel®, a nonabsorbed calcium- and aluminum-free phosphate binder, lowers serum phosphorus and parathyroid hormone," Kid Int. 55: 299-307 (1999).

### Protocol 6:

Chertow, G.M., Burke, S.K., Dillon, M.A., and Slatopolsky, E., for the RenaGel Study Group, "Long-term effects of sevelamer hydrochloride on the calcium x phosphorus product and lipid profile of haemodialysis patients," Nephol Dial Transplant. 14: 2907-2914 (1999).

### Results

Decreases in uric acid from the baseline to the end of the trial were found in all haemodialysis patient studies, as shown in Table 1.

**Table 1: Serum Uric Acid in Haemodialysis Patients Treated**

| Protocol | Baseline mg/dL | Endpoint | Change | P-Value |
|---|---|---|---|---|
| 1 | 5.5 | 5.3 | -0.3 | 0.3693 |
| 2 | 6.5 | 6.2 | -0.4 | 0.0622 |
| 3 | 6.7 | 6.2 | -0.7 | 0.0096 |
| 4 | 6.9 | 6.4 | -0.4 | 0.0001 |
| 5 | 7.4 | 6.8 | -0.7 | <0.0001 |
| 6 | 7.1 | 6.3 | -0.8 | <0.0001 |

Analysis of the database suggests that patients with a history of gout, especially those with hyperuricemia, had reductions in serum uric acid levels while being treated according to an embodiment of the invention.

## Claims

1. Use of a therapeutically effective amount of at least one amine polymer that binds to uric acid or to a precursor thereof for the manufacture of a medicament for treating gout in an individual in need thereof, wherein said polymer is **characterized by** a repeat unit having a formula selected from the group consisting of: 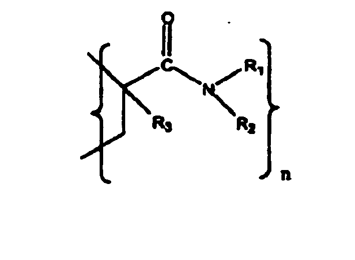 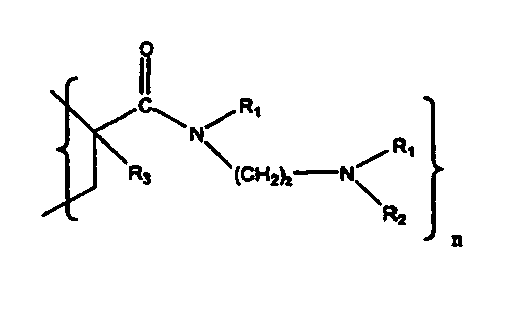 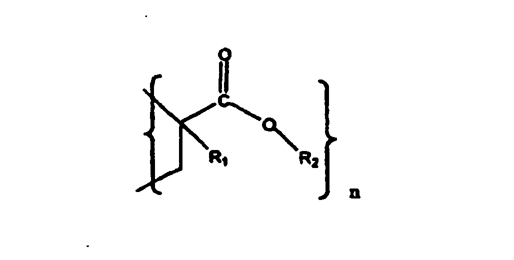 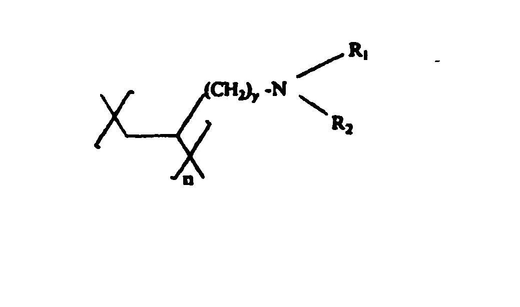 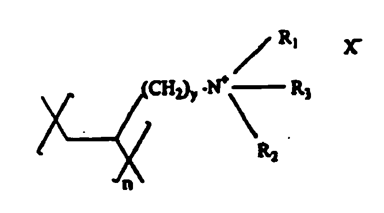 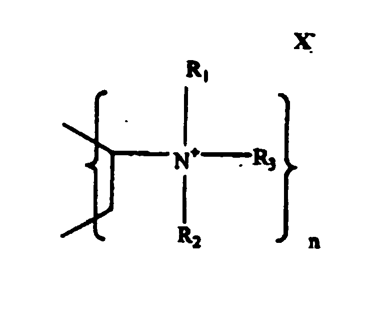 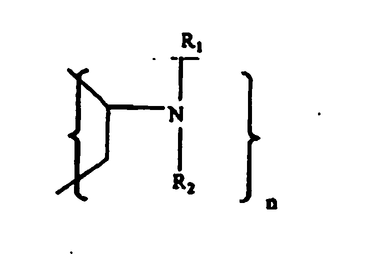 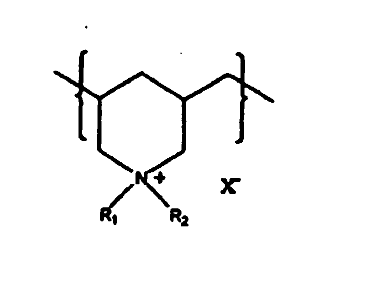 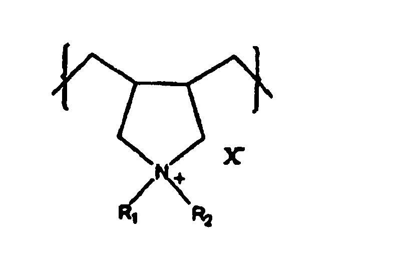 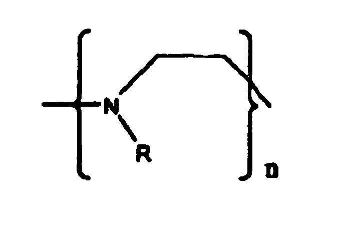 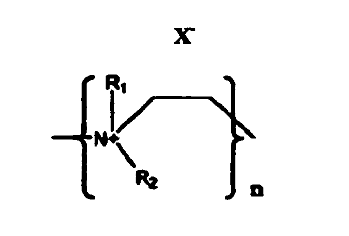 and salts and copolymers thereof, where n is a positive integer and y and z are integers of one or more, and R, R₁, R₂ and R₃, independently, is H or a substituted or unsubstituted alkyl group, alkylamino or aryl group and X⁻ is an exchangeable negatively charged counterion.

2. Use according to Claim 1 wherein the polymer that binds to uric acid is an aliphatic amine polymer.

3. Use according to Claim 1 or 2 wherein the polymer that binds to uric acid or to a precursor thereof is substituted by substituents, selected from the group consisting of amines, cyano groups, olefins, phosphines, arsines, sulfides, dithiocarbamates, nitrates, carboxylates, phenolates, acetylacetonates, and hydroxy groups.

4. Use according to Claim 1 wherein said polymer is cross-linked by means of a multifunctional cross-linking agent.

5. Use according to Claim 4 wherein the multifunctional cross-linking agent is present in an amount from 0.5-25% by weight, preferably in an amount from 2,5-20% by weight, based upon the combined weight of monomer and cross-linking agent.

6. Use according to Claim 4 wherein said cross-linking agent comprises epichlorohydrin.

7. Use according to Claim 4 wherein the polymer is a homopolymer.

8. Use according to Claim 4 wherein the polymer is selected from the group consisting of polyallylamines, polydiallylamines, and polyvinylamines.

9. Use according to Claim 1 wherein at least one of R, R₁, R₂, and R₃ in each formula is hydrogen.

10. Use according to Claim 1 wherein the medicament is to be administered with one or more meals.

11. Use of a therapeutically effective amount of at least one amine polymer that binds to uric acid or to a precursor thereof for the manufacture of a medicament for treating hyperuricosuria or uric acid nephropathy in an individual in need thereof, wherein said polymer is **characterized by** a repeat unit having a formula selected from the group consisting of: 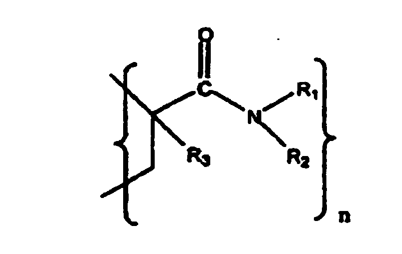 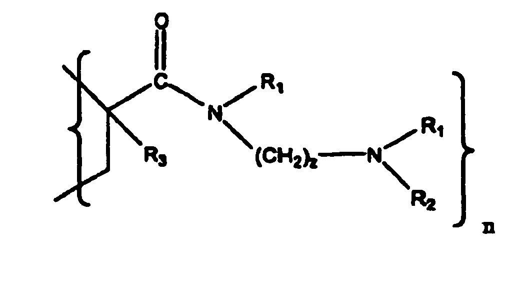 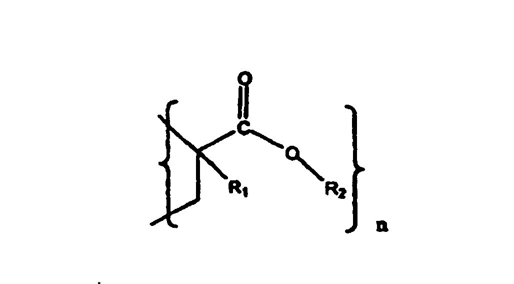 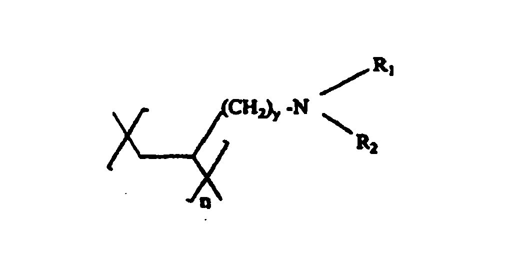 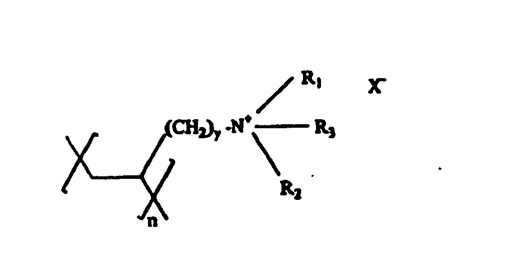 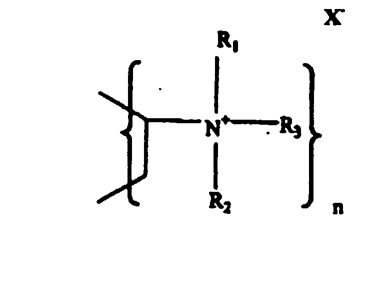 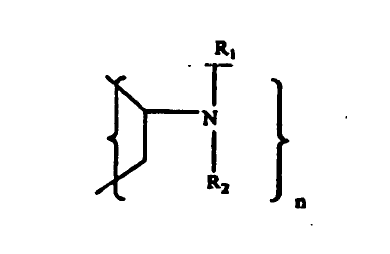 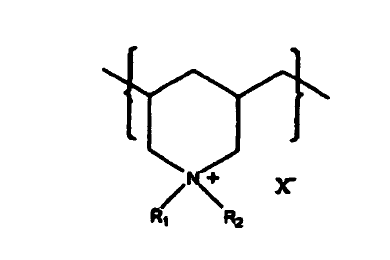 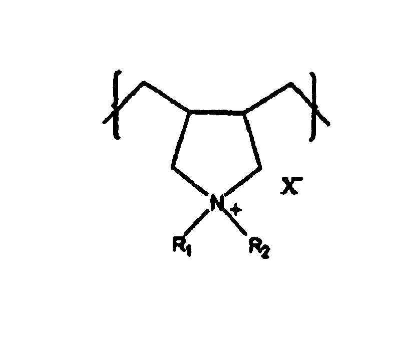 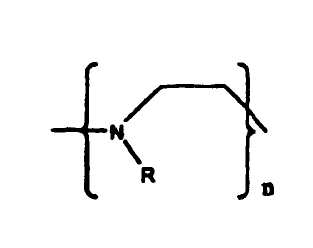 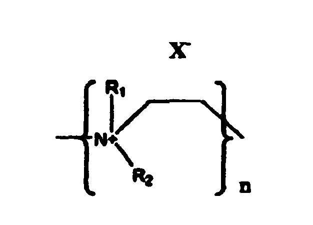 and salts and copolymers thereof, where n is a positive integer and y and z are integers of one or more, and R, R₁, R₂, and R₃, independently, is H or a substituted or unsubstituted alkyl, alkylamino or aryl group and X⁻ is an exchangeable negatively charged counterion.

12. Use according to Claim 11 wherein the polymer that binds to uric acid is an aliphatic amine polymer.

13. Use according to Claim 11 or 12 wherein the polymer that binds to uric acid is substituted by substituents selected from the group consisting of amines, cyano groups, olefins, phosphines, arsines, sulfides, dithiocarbamates, nitrates, carboxylates, phenolates, acetylacetonates, and hydroxy groups.

14. Use according to Claim 11 wherein said polymer is cross-linked by means of a multifunctional cross-linking agent.

15. Use according to Claim 14 wherein the multifunctional cross-linking agent, is present in an amount from 0.5-25% by weight, preferably in an amount from 2.5-20% by weight, based upon the combined weight of monomer and cross-linking agent.

16. Use according to Claim 14 wherein said cross-linking agent comprises epichlorohydrin.

17. Use according to Claim 14 wherein the polymer is a homopolymer.

18. Use according to Claim 14 wherein the polymer is selected from the group consisting of polyallylamines, polydiallylamines and polyvinylamines.

19. Use according to Claim 11 wherein at least one of R, R₁, R₂, and R₃ in each formula is hydrogen.

20. Use according to Claim 11, the medicament further comprising a nonsteroidal anti-inflammatory drug.

21. Use according to Claim 20 wherein the nonsteroidal anti-inflammatory drug includes colchicine.

22. Use according to Claim 11, the medicament further comprising a uric add synthesis inhibitor.

23. Use according to Claim 22 wherein the uric acid synthesis inhibitor includes a xanthine oxidase inhibitor.

24. Use according to Claim 23 wherein the xanthine oxidase inhibitor includes allopurinol.

25. Use according to Claim 11, the medicament further comprising a uricosuric agent.

26. Use according to Claim 11 wherein the medicament is to be administered of with one or more meals.

## Patentansprüche

1. Verwendung einer therapeutisch wirksamen Menge mindestens eines Aminpolymers, das an Harnsäure oder einen Vorläufer davon bindet, zur Herstellung eines Medikaments für die Behandlung von Gicht bei einem Individuum, welches derer bedarf, wobei das Polymer **gekennzeichnet ist durch** eine Struktureinheit mit einer Formel, ausgewählt aus der Gruppe, bestehend aus: 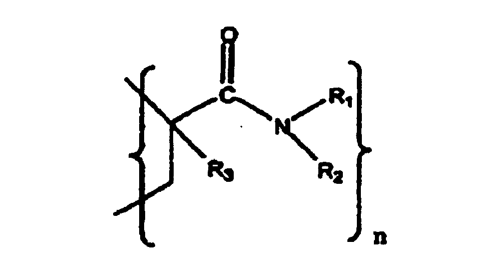 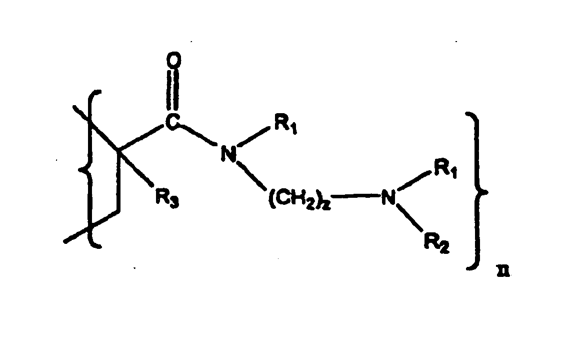 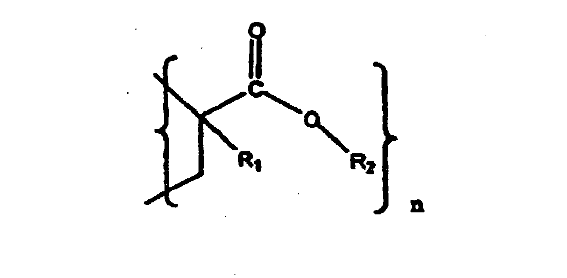 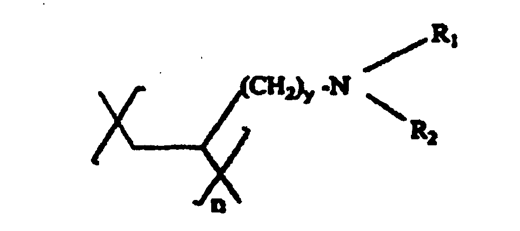 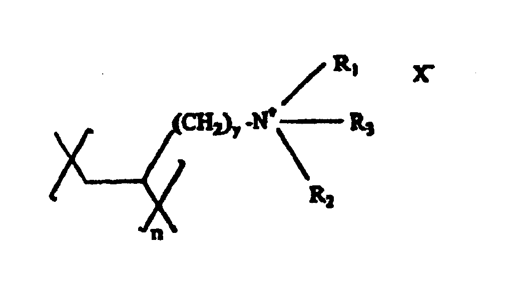 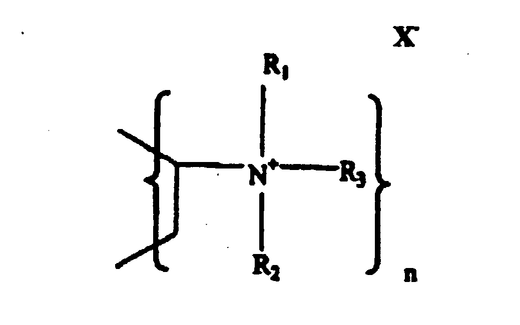 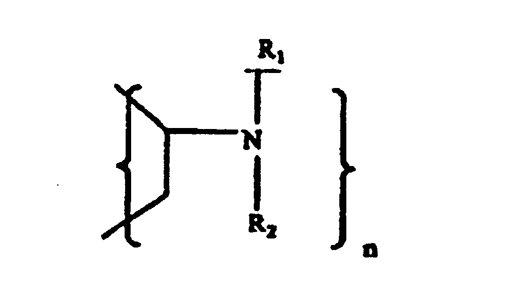 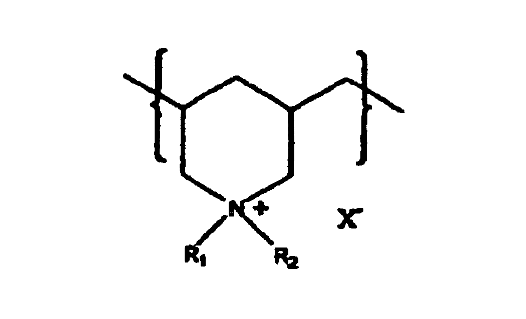 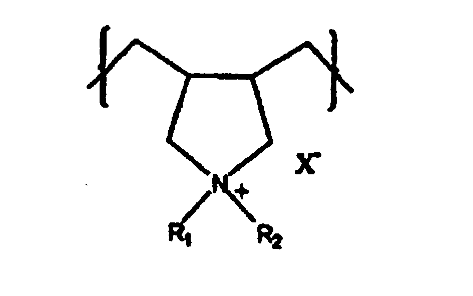 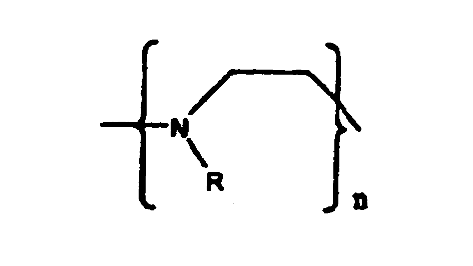 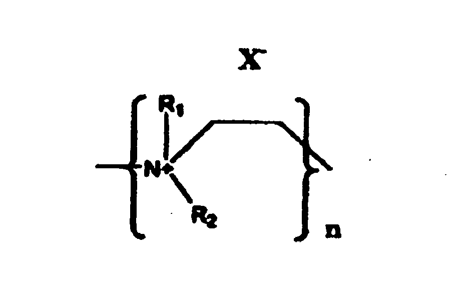 und von Salzen und Copolymeren davon, wobei n eine positive ganze Zahl darstellt und y und z ganze Zahlen von Eins oder darüber darstellen, und wobei R, R₁, R₂ und R₃ unabhängig voneinander H oder eine substituierte oder unsubstituierte Alkylgruppe, Alkylamino- oder Arylgruppe sind, und X⁻ ein austauschbares, negativ geladenes Gegenion ist.

2. Verwendung nach Anspruch 1, wobei das Polymer, das an Harnsäure bindet, ein aliphatisches Aminpolymer ist.

3. Verwendung nach Anspruch 1 oder 2, wobei das Polymer, das an Harnsäure oder einen Vorläufer davon bindet, durch Substituenten, ausgewählt aus der Gruppe, bestehend aus Aminen, Cyanogruppen, Olefinen, Phosphinen, Arsinen, Sulfiden, Dithiocarbamaten, Nitraten, Carboxylaten, Phenolaten, Acetylacetonaten und Hydroxygruppen, substituiert ist.

4. Verwendung nach Anspruch 1, wobei das Polymer mittels eines multifunktionellen Vernetzungsmittels vernetzt ist.

5. Verwendung nach Anspruch 4, wobei das multifunktionelle Vernetzungsmittel in einer Menge von 0,5-25 Gew.-%, bevorzugt in einer Menge von 2,5-20 Gew.-%, bezogen auf das kombinierte Gewicht von Monomer und Vernetzungsmittel, vorhanden ist.

6. Verwendung nach Anspruch 4, wobei das Vernetzungsmittel Epichlorhydrin umfasst.

7. Verwendung nach Anspruch 4, wobei das Polymer ein Homopolymer ist.

8. Verwendung nach Anspruch 4, wobei das Polymer ausgewählt wird aus der Gruppe, bestehend aus Polyallylaminen, Polydiallylaminen und Polyvinylaminen.

9. Verwendung nach Anspruch 1, wobei in jeder Formel mindestens einer der Reste R, R₁, R₂ und R₃ Wasserstoff ist.

10. Verwendung nach Anspruch 1, wobei das Medikament mit einer oder mehreren Mahlzeiten zu verabreichen ist.

11. Verwendung einer therapeutisch wirksamen Menge mindestens eines Aminpolymers, das an Harnsäure oder einen Vorläufer davon bindet, zur Herstellung eines Medikaments für die Behandlung von Hyperurikosurie oder Harnsäurenephropathie bei einem Individuum, welches derer bedarf, wobei das Polymer **gekennzeichnet ist durch** eine Struktureinheit mit einer Formel, ausgewählt aus der Gruppe, bestehend aus: 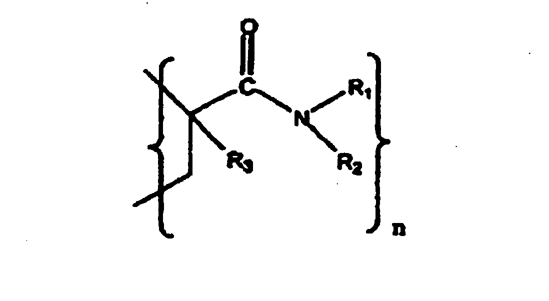 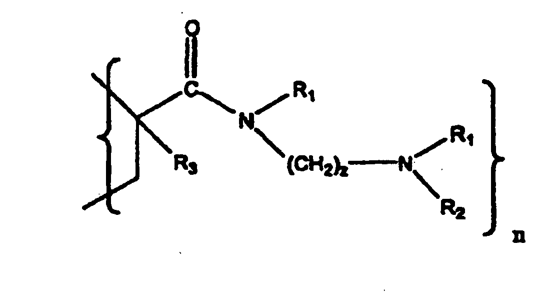 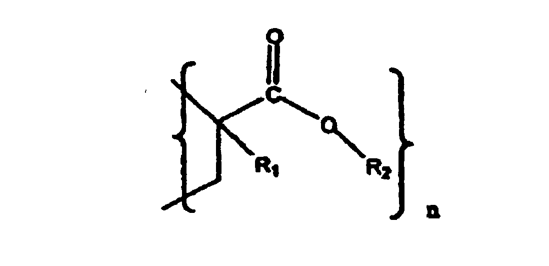 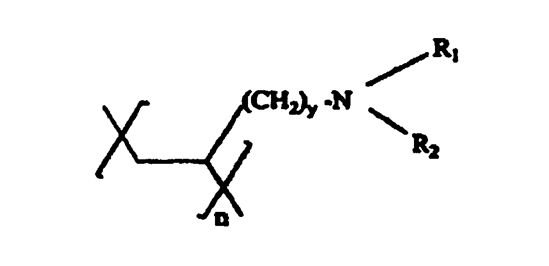 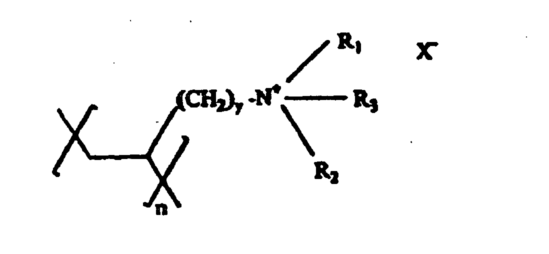 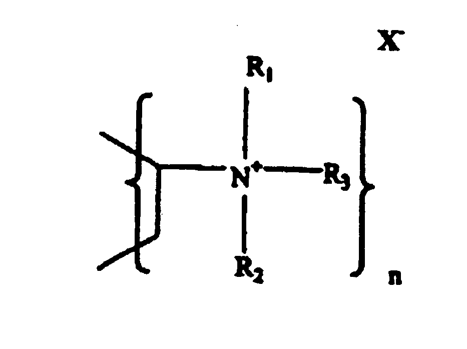 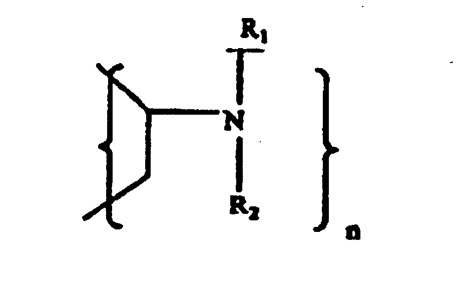 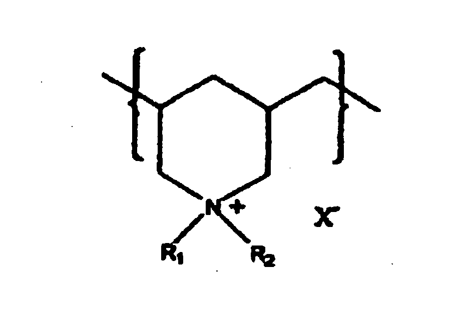 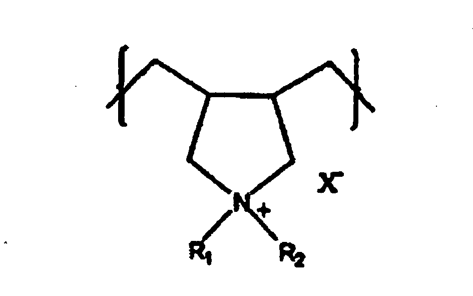 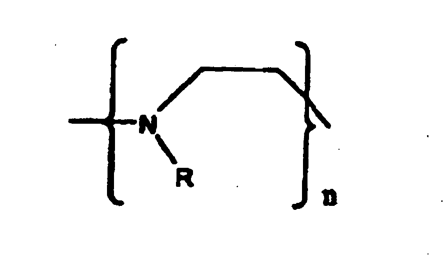 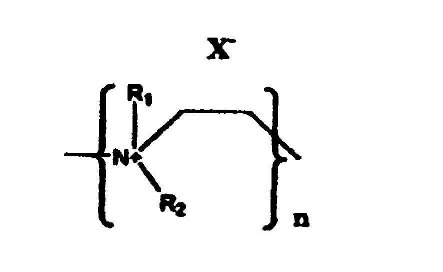 und von Salzen und Copolymeren davon, wobei n eine positive ganze Zahl darstellt und y und z ganze Zahlen von Eins oder darüber darstellen, und wobei R, R₁, R₂ und R₃ unabhängig voneinander jeweils H oder eine substituierte oder unsubstituierte Alkyl-, Alkylamino- oder Arylgruppe sind, und X⁻ ein austauschbares, negativ geladenes Gegenion ist.

12. Verwendung nach Anspruch 11, wobei das Polymer, das an Harnsäure bindet, ein aliphatisches Aminpolymer ist.

13. Verwendung nach Anspruch 11 oder 12, wobei das Polymer, das an Harnsäure bindet, durch Substituenten, ausgewählt aus der Gruppe, bestehend aus Aminen, Cyanogruppen, Olefinen, Phosphinen, Arsinen, Sulfiden, Dithiocarbamaten, Nitraten, Carboxylaten, Phenolaten, Acetylacetonaten und Hydroxygruppen, substituiert ist.

14. Verwendung nach Anspruch 11, wobei das Polymer mittels eines multifunktionellen Vernetzungsmittels vernetzt ist.

15. Verwendung nach Anspruch 14, wobei das multifunktionelle Vernetzungsmittel in einer Menge von 0,5-25 Gew.-%, bevorzugt in einer Menge von 2,5-20 Gew.-%, bezogen auf das kombinierte Gewicht von Monomer und Vernetzungsmittel, vorhanden ist.

16. Verwendung nach Anspruch 14, wobei das Vernetzungsmittel Epichlorhydrin umfasst.

17. Verwendung nach Anspruch 14, wobei das Polymer ein Homopolymer ist.

18. Verwendung nach Anspruch 14, wobei das Polymer ausgewählt wird aus der Gruppe, bestehend aus Polyallylaminen, Polydiallylaminen und Polyvinylaminen.

19. Verwendung nach Anspruch 11, wobei in jeder Formel mindestens einer der Reste R, R₁, R₂ und R₃ Wasserstoff ist.

20. Verwendung nach Anspruch 11, wobei das Medikament weiterhin ein nichtsteroidales entzündungshemmendes Arzneimittel umfasst.

21. Verwendung nach Anspruch 20, wobei das nichtsteroidale entzündungshemmende Arzneimittel Colchicin umfasst.

22. Verwendung nach Anspruch 11, wobei das Medikament weiterhin einen Inhibitor der Harnsäuresynthese umfasst.

23. Verwendung nach Anspruch 22, wobei der Inhibitor der Harnsäuresynthese einen Xanthinoxidase-Inhibitor umfasst.

24. Verwendung nach Anspruch 23, wobei der Xanthinoxidase-Inhibitor Allopurinol umfasst.

25. Verwendung nach Anspruch 11, wobei das Medikament weiterhin ein urikosurisches Mittel umfasst.

26. Verwendung nach Anspruch 11, wobei das Medikament mit einer oder mehreren Mahlzeiten zu verabreichen ist.

## Revendications

1. Utilisation d'une quantité thérapeutiquement efficace d'au moins un polymère aminé qui se lie à l'acide urique ou à un précurseur de celui-ci pour la fabrication d'un médicament permettant le traitement de la goutte chez un individu qui en a besoin, ledit polymère étant **caractérisé par** une unité répétitive ayant une formule sélectionné dans le group constitué de: 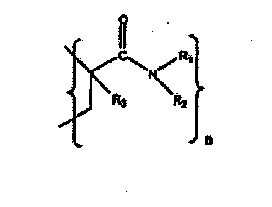 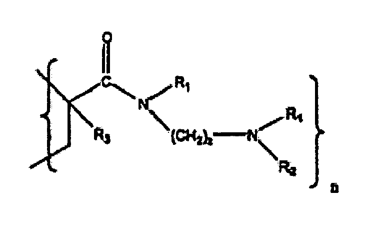 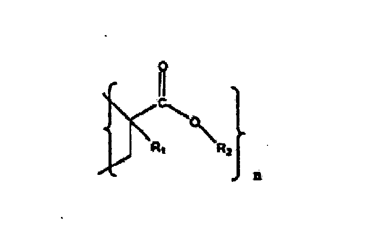 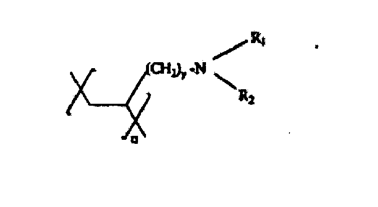 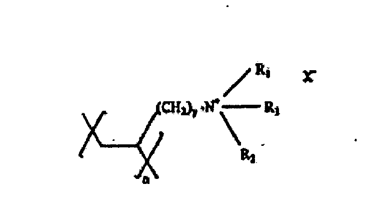 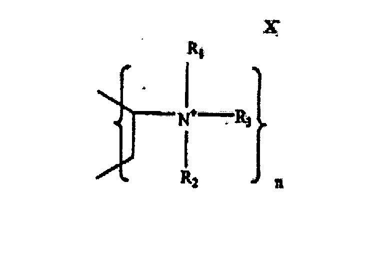 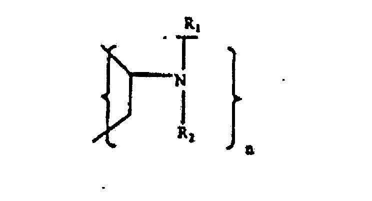 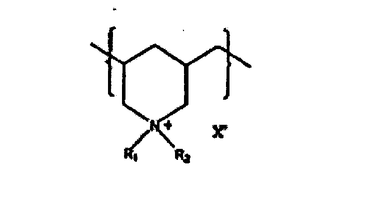 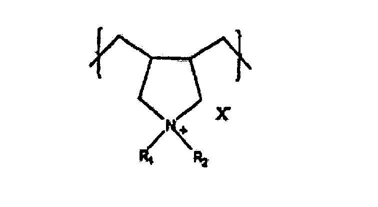 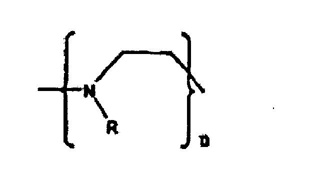 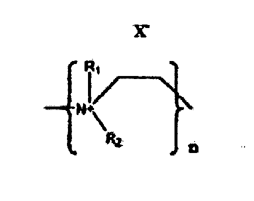 et de sels et de copolymères de ceux-ci, n étant un entier positif et y et z étant des entiers de un ou supérieurs à un, et R, R₁, R₂ et R₃ étant indépendamment H ou un groupe alkyle substitué ou non substitué, un groupe alkylamino ou aryle et X⁻ étant un ion antagoniste échangeable chargé négativement.

2. Utilisation selon la revendication 1, dans laquelle le polymère qui se lie à l'acide urique est un polymère aminé aliphatique.

3. Utilisation selon la revendication 1 ou 2, dans laquelle le polymère qui se lie à l'acide urique ou à un précurseur de celui-ci est substitué par des substituants sélectionnés dans le groupe constitué d'amines, de groupes cyano, d'oléfines, de phosphines, d'arsines, de sulfures, de dithiocarbamates, de nitrates, de carboxylates, de phénolates, d'acétylacétonates et de groupes hydroxy.

4. Utilisation selon la revendication 1, dans laquelle ledit polymère est réticulé à l'aide d'un agent de réticulation multifonctionnel.

5. Utilisation selon la revendication 4, dans laquelle l'agent de réticulation multifonctionnel est présent dans une quantité de 0,5 à 25% en poids, de préférence dans une quantité de 2,5 à 20% en poids sur la base du poids combiné du monomère et de l'agent de réticulation.

6. Utilisation selon la revendication 4, dans laquelle ledit agent de réticulation comprend l'épichlorohydrine.

7. Utilisation selon la revendication 4, dans laquelle le polymère est un homopolymère.

8. Utilisation selon la revendication 4, dans laquelle le polymère est sélectionné dans le groupe constitué de polyallylamines, de polydiallylamines et de polyvinylamines.

9. Utilisation selon la revendication 1, dans laquelle au moins parmi R, R₁, R₂ et R₃ dans chaque formule est de l'hydrogène.

10. Utilisation selon la revendication 1, dans laquelle le médicament doit être administré pendant un ou plusieurs repas.

11. Utilisation d'une quantité thérapeutiquement efficace d'au moins un polymère aminé qui se lie à l'acide urique ou à un précurseur de celui-ci pour la fabrication d'un médicament permettant le traitement de l'hyperuricosurie ou une néphropathie urique chez un individu qui en a besoin, dans laquelle ledit polymère est **caractérisé par** une unité répétitive ayant une formule sélectionnée dans le group constitué de: 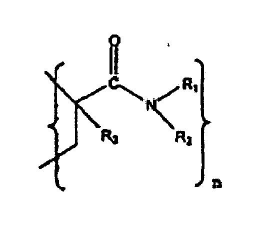 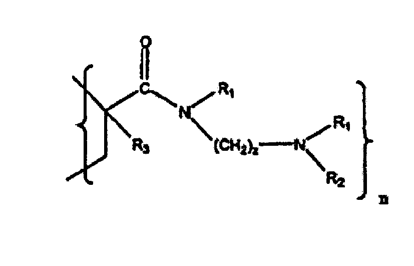 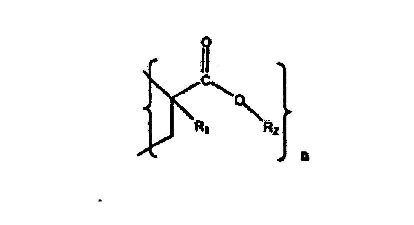 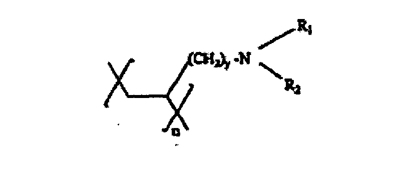 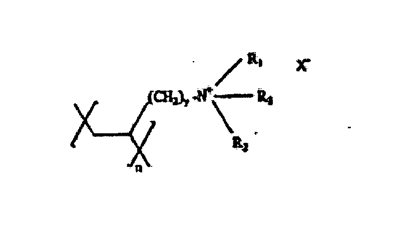 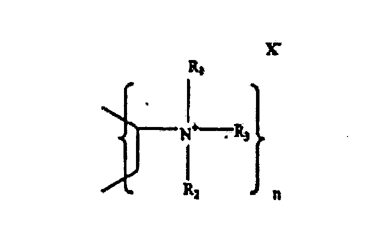 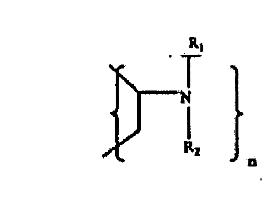 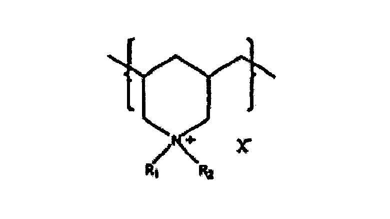 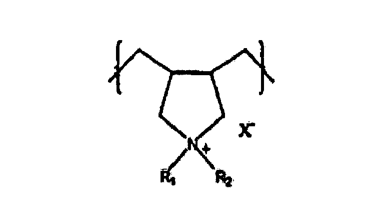 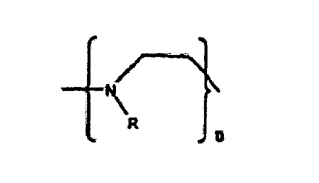 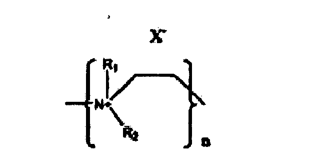 et de sels et de copolymères de ceux-ci, n étant un entier positif et y et z étant des entiers de un ou supérieurs à un, et R, R₁, R₂ et R₃ étant indépendamment H ou un groupe alkyle substitué ou non substitué, un groupe alkylamino ou aryle et X⁻ étant un ion antagoniste échangeable chargé négativement.

12. Utilisation selon la revendication 11, dans laquelle le polymère qui se lie à l'acide urique est un polymère aminé aliphatique.

13. Utilisation selon la revendication 11 ou 12, dans laquelle le polymère qui se lie à l'acide urique est substitué par des substituants sélectionnés dans le groupe constitué d'amines, de groupes cyano, d'oléfines, de phosphines, d'arsines, de sulfures, de dithiocarbamates, de nitrates, de carboxylates, de phénolates, d'acétylacétonates et de groupes hydroxy.

14. Utilisation selon la revendication 11, dans laquelle ledit polymère est réticulé à l'aide d'un agent de réticulation multifonctionnel.

15. Utilisation selon la revendication 14, dans laquelle l'agent de réticulation multifonctionnel est présent dans une quantité de 0,5 à 25% en poids, de préférence dans une quantité de 2,5 à 20% en poids sur la base du poids combiné du monomère et de l'agent de réticulation.

16. Utilisation selon la revendication 14, dans laquelle ledit agent de réticulation comprend l'épichlorohydrine.

17. Utilisation selon la revendication 14, dans laquelle le polymère est un homopolymère.

18. Utilisation selon la revendication 14, dans laquelle le polymère est sélectionné dans le groupe constitué de polyallylamines, de polydiallylamines et de polyvinylamines.

19. Utilisation selon la revendication 11, dans laquelle au moins parmi R, R₁, R₂ et R₃ dans chaque formule est de l'hydrogène.

20. Utilisation selon la revendication 11, le médicament comprenant en outre une drogue anti-inflammatoire non stéroïdienne.

21. Utilisation selon la revendication 20 dans laquelle la drogue anti-inflammatoire non stéroïdienne comprend la colchicine.

22. Utilisation selon la revendication 11, le médicament comprenant en outre un inhibiteur de la synthèse de l'acide urique.

23. Utilisation selon la revendication 22, dans laquelle l'inhibiteur de la synthèse de l'acide urique comprend un inhibiteur de xanthine oxydase.

24. Utilisation selon la revendication 22, dans laquelle l'inhibiteur de xanthine oxydase comprend l'allopurinol.

25. Utilisation selon la revendication 11, le médicament comprenant en outre un agent uricosurique.

26. Utilisation selon la revendication 11, dans laquelle le médicament doit être administré pendant un ou plusieurs repas.
